Europäisches Patentamt

European Patent Office

Office européen des brevets

(19)

(11) Veröffentlichungsnummer: **0 164 571**
**B1**

(12) **EUROPÄISCHE PATENTSCHRIFT**

(45) Veröffentlichungstag der Patentschrift: 27.06.90

(21) Anmeldenummer: 85105612.7

(22) Anmeldetag: 08.05.85

(51) Int. Cl.⁵: **A 61 K 9/22, A 61 K 31/165**

(54) Retardformen des alpha-(2,5-Dimethoxy-phenyl)-beta-lycinamido-äthanols und Verfahren zu ihrer Herstellung.

(30) Priorität: 11.05.84 DE 3417576

(43) Veröffentlichungstag der Anmeldung:
18.12.85 Patentblatt 85/51

(45) Bekanntmachung des Hinweises auf die
Patenterteilung:
27.06.90 Patentblatt 90/26

(84) Benannte Vertragsstaaten:
AT BE CH DE FR GB IT LI LU NL SE

(56) Entgegenhaltungen:
EP-A-0 108 882
DE-A-2 824 112
DE-B-1 493 917
FR-A-2 400 950

CHEMICAL ABSTRACTS, Band 99, 1983, Seite 314, Nr. 43465s, Columbus, Ohio, US; W. KORSATKO et al.: "Poly-D(-)-3-hydroxybutyric acid (PHBA) - a biodegradable carrier for long term medication dosage. 1. Development of parenteral matrix tablets for long term application of pharmaceuticals" & PHARM. IND. 1983, 45(5),525-7

(73) Patentinhaber: CL PHARMA
AKTIENGESELLSCHAFT
St. Peter-Strasse 25
A-4021 Linz (AT)
(84) BE CH DE FR GB IT LI LU NL SE AT

(72) Erfinder: Korsatko, Werner, Dr.
Kärntnerstrasse 432
A-8054 Graz (AT)
Erfinder: Korsatko-Wabnegg, Brigitta
Kärntnerstrasse 432
A-8054 Graz (AT)

(74) Vertreter: Kunz, Ekkehard, Dr.
Chemie Holding AG Patentwesen St. Peter-Strasse 25
A-4021 Linz (AT)

(56) Entgegenhaltungen:
Pharm. Ind. 45 Nr 5(1983), pp 525-527

EP 0 164 571 B1

## Beschreibung

Die Erfindung betrifft orale Retardformen des alpha-(2,5-Dimethoxy-phenyl)-β-glycinamidoäthanols (Midodrin) und ein Verfahren zu deren Herstellung. Diese Retardformulierungen sind duch Verpressen hergestellte Arzneimittel-Formkörper, die Poly-D(−)-3-hydroxybuttersäure mit einem bestimmten Molekulargewicht und einer ausgewählten Korngröße als Trägermaterial (Matrix) enthaltend und nach oraler Applikation den Wirkstoff im Magen-Darm-Trakt in geregelter Weise verzögert abgeben.

Bei Midodrin [alpha-(2,5-Dimethoxy-phenyl-β-glycinamidoäthanol] und seinem Hydrochlorid handelt es sich um einen seit Jahren bewährten Wirkstoff (DE—PS—1 493 917). Die therapeutischen Wirkungen von Midodrin-Hydrochlorid bei sympathotoner und asympathotoner Hypotonie wurden in klinischen Untersuchungen vielfach besätigt. Midodrin-Hydrochlorid wirkt zum Unterschied von anderen bekannten Symapthomimetika, die auf alpha- und β-Rezeptoren ansprechen, selektiv auf die peripheren alpha-Rezeptoren und findet seinen Anwendungsbereich in der Therapie von konstitutioneller und symptomatischer Hyptonie. Anwendungsgebiete sind beispielsweise hypotone Zustände bei Infektionen, in der Rekonvaleszenz, bei Operationen und Entbindungen, weiters hypotone Labilität bei Wetterfühligkeit und Föhnbeschwerden oder morgendliche Startbeschwerden. Bei diesen Krankheitsbildern leiden die Patienten bekanntermaßen unmittelbar nach dem Aufstehen in den ersten Morgenstunden besonders stark und den Symptomen der Hypotonie wie Schwindel- und Schwächegefühlen, Kopfschmerzen, Antriebslosigkeit und so weiter.

Deshalb würden Retardformen von Midodrin-Hydrochlorid, welche den Wirkstoff nach der Einnahme des Medikaments am Vorabend so gleichmäßig und verzögert abgeben, daß therapeutische Blutspiegelwerte trotz des großen Einnahmeintervalls über Nacht bis zum nächsten Morgen aufrecht erhalten werden könnten, große Vorteile bieten, da dem Patienten auf diese Weise ein beschwerdefreies Aufstehen ermöglicht würde.

Neuere Untersuchungen haben zudem ergeben, daß die Verabreichung von Midodrin-Hydrochlorid für Patienten, die unter schwersten Formen von Kreislaufregulationsstörungen und -fehlregulationen, wie beispielsweise der idiopathischen orthostatischen Hypotonie oder dem Shy-Drager-Syndrom, leiden, in vielen Fällen die einzige bisher bekannte wirksame medikamentöse Behandlung darstellt. (vgl. dazu Midodrine, a new agent in the management of idiopathic orthostatic hypotention and Shy-Dragersyndrome von Alexander Schirger et al., erschienen Mayo Clin. Proc. Juli 1981, Vol. 56, Seiten 429—433). Da Patienten mit diesen chronischen Krankheitsbildern von einer Dauerbehandlung mit Midodrin-Hydrochlorid abhängig sind, würde eine Verringerung der Zahl der Einnahmen pro Tag die Lebenssituation dieser Patienten verbessern, was gerade bei einer Langzeitmedikation anerkanntermaßen von Bedeutung ist.

Aufgrund dieser offensichtlichen Vorteile einer Retardform von Midodrin-Hydrochlorid hat es nicht an Versuchen zu ihrer Realiserung gefehlt. Pharmazeutische Retardformen können beispielsweise dadurch zubereitet werden, daß der Wirkstoff zusammen mit Hilfsstoffen so formuliert wird, daß er langsam freigegeben wird, z.B. durch Einbetten in eine sich nicht oder nur langsam auflösende Matrix. Bei der Anwendung dieser Methode auf Midodrin-Hydrochlorid hat sich nun gezeigt, daß man mit bekannten Matrixsystemen wie mit Cellulosederivaten, Polyvinylacetat, Polyäthylen, Polyvinylchlorid oder Polymethacrylat zwar eine gute Retardierung des Wirkstoffs erreichen kann, aber dafür den Nachteil in Kauf nehmen muß, daß bei solchen Midodrin-Hydrochlorid-Retardformen die Freisetzung der Initialdosis in der Anfangsphase der Medikation viel zu langsam erfolgt.

In W. Korsatko et al., Pharm. Ind. 1983, 45(5), 525—527 ist beschrieben, daß Implantationstabletten, die aus Poly-HB und dem Wirkstoff 7-Hydroxyethyltheophyllin bestehen, gute Retardierungseigenschaften besitzen. Beim Versuch, Retardtabletten aus Poly-HB und Midodrin nach W. Korsatko herzustellen, trat jedoch ebenfalls das Problem auf, daß die Freisetzung des Wirkstoffes zu stark verzögert wurde. Dieser Nachteil ließe sich nur durch die Bereitstellung von aufwendigeren Arzneiformen verringern, bei denen die als Initialdosis benötigte Wirkstoffmenge in der Instantform nachträglich auf die Retardform, beispielsweise durch Aufsprühen, aufgebracht wird. Die Herstellung solcher gemischter Arzneiformen ist aber technisch sehr aufwendig und führt regelmäßig zu nicht unerheblichen Wirkstoffverlusten.

Es ist nun unerwarteterweise gelungen, Midodrin-Hydrochlorid-Retardformen zu finden, bei denen eine ausreichende Initialdosis nach der Verabreichung direkt aus einer einheitlichen Retardform freigesetzt und ein lang anhaltender gleichmäßiger Midodrin-Hydrochlorid-Blutspiegel gewährleistet wird. Gleichzeitig haben diese Retardformen noch den Vorteil, daß sie auf einfache Weise in Form von üblichen oralen Arzneiformen z.B. Tabletten hergestellt werden können und bei der Arzneiformengebung die oben skizzierten Nachteile von bekannten Matrixformen nicht auftreten.

Die erfindungsgemäße Lösung des Problems besteht darin, daß man Midodrin-Hydrochlorid als Wirkstoff in einem bestimmten Gewichtsverhältnis mit einer auf mikrobiellem Weg durch Fermentation gewonnenen Poly-D(−)-3-hydroxybuttersäure mit einem genügend hohen Molekulargewicht und einer bestimmten, ausgewählten Korngrößenfraktion vermischt und zu Preßlingen verarbeitet.

Gegenstand der Erfindung sind demnach orale Retardformen des alpha-(2,5-Dimethoxy-phenyl)-beta-glycinamidoäthanols (Midodrin) in Form von festen, durch Verpressen hergestellten Arzneimittel-Formkörpern, bestehend aus dem Wirkstoff, einer auf mikrobiellen Weg gewonnenen Poly-D(−)-3-hydroxybuttersäure (PHB) und gegebenenfalls in der Galenik üblichen Hilfs- und Zusatzstoffen, dadurch

2

gekennzeichnet, daß die Retardform auf einen Gew. Teil alpha-(2,5-Dimethoxy-phenyl)-beta-glycinamidoäthanol in Form des Hydrochlorids (Midodrin-Hydrochlorid) 1 bis 15 Gew. Teile Poly-D-(−)-3-hydroxybuttersäure mit einem Molekulargewicht von mindestens 50000 und einer ausgewählten Korngrößenfraktion im Bereich von 0.05 bis 0.6 mm in homogener Mischung enthält.

Als Arzneimittel-Formkörper für die erfindungsgemäßen Retardformen kommen Preßlinge in jeder Größe und Form, die für die orale Applikation geeignet sind, in Betracht, wie Tabletten, Drageekerne, Stäbchen und so weiter. Jedoch eignen sich für diesen Zweck auch andere durch Verpressen hergestellte Arzneiformen, beispielsweise zerkleinerte Komprimate, die anschließend in Kapseln abgefüllt werden, oder auch Mehrschichttabletten oder -dragees, bei denen neben der Retardschicht in einer anderen Schicht nichtretardierte Wirkstoffe enthalten sein sollen. Besonders bevorzugte Arzneiformen für die erfindungsgemäßen Retardpräparate sind Tabletten, die auf einfache Weise in einem einzigen Arbeitsgang hergestellt werden können und eine für den Patienten vertraute und dadurch besonders gern angenommene Arzneiform darstellen. Diese Tabletten können eine oder mehrere Bruchrillen aufweisen, die es dem Patienten ermöglichen die Tablette zu zerkleinern und das Präparat über die angebotenen Dosierungseinheiten hinaus in der vom Arzt verordneten oder nach eigener Erfahrung gewählten, individuellen Dosis zu sich zu nehmen.

Die als Arzneimittelträger und Retardierungsmittel verwendete Poly-D(−)-3-hydroxybuttersäure (PHB) wird auf fermentativem Weg aus dem Zellmaterial von Mikroorganismen prokaryotischer Natur, welche imstande sind PHB zellintern als Speicherstoff für Energie und Kohlenstoff anzuhäufen, gewonnen. PHBspeichernde Mikroorganismen sind inzwischen in etwa 50 verschiedenen Arten von Bakterien bei fast 150 verschiedenen Stämmen nachgewiesen worden und finden sich beispielsweise bei phototrophen Bakterien, gramnegativen aeroben Stäbchen und Kokken, gramnegativen Kokken und Kokkobacillen, gramnegativen chemolithothrophen Bakterien, Endosporen bildenden Stäbchen und Kokken, grampositiven asporogenen stäbchenförmigen Bakterien, methylotrophen Bakterien, Aktinomyceten, knospenden oder Anhängsel tragenden Bakterien, Cyanobakterien. Die chemische Struktur einer durch Fermentation und Isolierung aus mikrobieller Zellmasse gewonnenen PHB ist die eines sterisch einheitlichen, chiralen Polymeren, wobei etwa 600 bis 30000 Wiederholungseinheiten gefunden werden können. Für die erfindungsgemäßen Matrixformen eignen sich Polymere mit einem Molekulargewicht von mindestens 50000 bis etwa 2 000 000, wobei Polymer mit einem Molekulargewicht von mindestens 50000 bis etwa 1 500 000 besonders bevorzugt sind. Die fermentative Herstellung von PHB und Isolierung aus dem Zellmaterial wurde beispielweise von Lafferty et al. in Chem. Rundschau 30 (41), 14 bis 16, (1977) beschrieben.

Ein großer Vorteil der Erfindung liegt darin, daß sich die Freisetzungsgeschwindigkeit des Wirkstoffs aus der Retardform beim erfindungsgemäßen Matrixsystem über den gewünschten Zeitraum von mehreren Stunden praktisch beliebig einstellen läßt. Der Retardeffekt ist überrachenderweise einerseits von der Korngröße der PHB und andererseits vom Gewichtsverhältnis der PHB-Matrix zum Wirkstoff, Midodrin-Hydrochlorid, abhängig. Diese Parameter werden zur Hebeiführung einer optimalen Wirkung bei der Behandlung der Hyptonie mit den erfindungsgemäßen Midodrin-Hydrochlorid-Retardformen so gewählt, daß

a) durch Freisetzung einer ausreichenden Initialdosis innerhalb der ersten 20—60 Minuten nach der Verabreichung therapeutisch wirksame Blutspiegelwerte aufgebaut werden und

b) zur Erhaltung der Blutspiegelwerte innerhalb des physiologischen Zeitraums für die Magen-Darmpassage des Präparats von etwa 4—12 Stunden, mit einem Optimum von etwa 6—10 Stunden, eine möglichst vollständige Freisetzung des Wirkstoffs unabhängig vom pH-Wert im Magen-Darmtrakt gewährleistet ist.

Midodrin-Hydrochlorid-Retardpräparate mit Freisetzungsraten, die den oben in Punkt a) und b) angegebenen Erfordernissen in jeder Weise genügen, werden unerwarteterweise nur dann erhalten, wenn man zur Retardierung als polymeres Trägermaterial eine Poly-D(−)-3-hydroxybuttersäure mit einer Korngröße von 0.05 bis 0.6 mm, vorzugsweise mit einer Korngröße von 0.2 bis 0.5 mm, verwendet und solche Mischungen aus polymerer Matrix und Wirkstoff hergestellt, die auf 1 Gew. Teil 1 bis 15 Gew. Teile, vorzugsweise jedoch 2 bis 6 Gew. Teile, ganz bevorzugt 3 bis 5 Gew. Teile PHB enthalten. Diese Mischungen werden dann zu den erfindungsgemäßen Depot-Arzneiformen verpreßt. Mit Korngrößenfraktionen, die kleiner als 0.05 mm sind, wird bei gleichen Gewichtsverhältnissen die Freisetzung des Wirkstoffs über den physiologischen Zeitraum hinaus zusätzlich verzögert. Bei Korngrößenfraktionen über 0.6 mm wird in vielen Fällen beim Preßvorgang Entmischung beobachtet, worunter die Verarbeitungsmöglichkeiten und die Dosierenauigkeit leidet.

Ist in schweren oder pathologischen Fällen von Hypotonie aus therapeutischen Gründen eine hohe Initialdosis innerhalb der ersten dreißig Minuten und eine hohe Erhaltungsdosis über 1 bis 4 Stunden notwenidg, so genügen diesem Erfordernis erfindungsgemäße Retardformen, welche auf 1 Gew. Teil Midodrin-Hydrochlorid 1 bis 2 Gew.Teile PHB enthalten. Erfordert die Therapie andererseits nach Freisetzung der Initialdosis die Aufrechterhaltung einer gleichmäßiger Erhaltungsdosis über mehrere Stunden, so sind für Depot-Arzneiformen, die innerhalb von 8 bis 10 Stunden den Wirkstoff vollständig abgeben sollen, Gewichtsverhältnisse Midodrin-Hydrochlorid: PHB von 1:3 bis 1:5 empfehlenswert. Ist ausnahmsweise ein Retardierungseffekt von mehr als 10 Stunden erwünscht, können auch Arzneiformen mit 6 bis 15 Gew.Teilen PHB eingesetzt werden.

Midodrin-Hydrochlorid ist ein sehr wirksamer Arzneistoff, der in ausgesprochen niedrigen Dosen dem Patienten verabreicht wird. Der Dosisbereich für die erfindungsgemäßen Retardformen liegt zwischen 3.5 und 20 mg Midodrin-Hydrochlorid pro Dosiseinheit, vorzugsweise jedoch zwischen 7.5 und 15 mg. In einzelnen Fällen pathologischer Krankheitsformen kann unter ärzlicher Kontrolle ausnahmsweise ein höherer Dosisbereich pro Dosiseinheit bei der Retardform angebracht sein.

Ein weiterer Gegenstand der Erfindung ist ein Verfahren zur Herstellung dieser Midodrin-Hydrochlorid-Retardformen.

Die durch Fermentation und Isolierung aus der Biomasse gewonnene PHB zeichnet sich im Gegensatz zu Polylactiden oder anderen für galenische Zwecke eingesetzten biologisch abbaubaren Polymeren durch günstige physikalische Eigenschaften wie geringe elastische Eigenshaften und geringe elektrostatische Auflaudungstendenz aus, und verfügt auch über gute Schmier- und Gleiteigenschaften. Daher, können die erfindungsgemäßen Retardformen sowohl in technischer als auch apparativer Hinsicht auf unerwartet einfache Art und Weise hergestellt werden.

Das Fermentationsprodukt wird durch Malen zerkleinert und aus dem Malgut wird dann durch Siebanalyse die für die Herstellung des Homogenats gewünschte Korngrößenfraktion im Bereich von 0.05 bis 0.6 mm gewonnen.

Im einzelnen geht man beim erfindungsgemäßen Verfahren sor vor, daß man den Wirkstoff auf mechanische Weise mit der PHB im gewünschten Gewichtsverhältnis vermischt und in eine homogene Form bringt.

Das Homogenisieren kann bei geringen Materialmengen zweckmäßigerweise mittels Pulverreibschale und Pistill oder einer Pulvermischdose erfolgen. Große Ansätze lassen sich vorteilhafterweise mit Hilfe von rotierenden Trommeln, Kubusmischern, Schaufelmischern, Tellermischern, Mischschnecken, Ribbonmischern, Konusmischern, Doppelkonusmischern, V-Mischern (Twin-Shell-blenden) oder ähnlichen Geräten homogenisieren.

Die auf diese Weise erhaltenen Homogenate werden anschließend gemäß einer bevorzugten Verfahrensmethode direkt ohne weitere Zuschlagstoffe zu Tabletten, Drageekernen, Stäbchen oder anderen Komprimaten beliebiger Form verpreßt. Der Wirkstoff läßt sich aber auch mit PHB und gewünschtenfalls weiteren direkttablettierbaren Hilfsstoffen, Schmiermitteln uzw. zu einer direkttablettierbaren Mischung verarbeiten, die dann zu Arzneimittel-Formkörpern verpreßt sind.

Die Herstellung der erfindungsgemäßen Arzneimittelformkörper ist mit allen herkömmlichen, manuellen oder automatischen Pressen möglich. Der Preßdruck kann über den Bereich von 0.1 bis etwa 10 Tonnen/cm², entsprechend 9.81 bis 981 N/mm² beliebig variiert werden und richtet sich je nach Art und Form der hergstellten Preßlinge. Die Freisetzungsrate des Wirkstoffs weist bei einer Variation des Preßdrucks im oben angegebenen Bereich keine signifikante Abhängigkeit vom Preßdruck auf.

Natürlich kann man auch das Homogenat aus Wirkstoff und PHB zuerst feucht oder trocken nach einem der üblichen Granulierverfahren zu einem Granulat verarbeiten, und diese Granulate gegebenenfalls nach Zumischung von in der Galenik üblichen Hilfsstoffen zu Arzneimittel-Formkörpern verpressen. Man kann aber auch den Wirkstoff gegebenenfalls mit üblichen Hilfsstoffen zuerst durch Feucht- und Trockengranulation in ein Granulat überführen, dem nachträglich die PHB zugesetzt wird, worauf die Masse zu Arzneimittel-Formkörpern verpreßt wird. Die Anwendung solcher Granulierverfahren beinhaltet einen zusätzlichen Verfahrensschritt, bietet aber gegenüber der Direktverpressung des Homogenats keinen nennenswerten Vorteil.

Die Einfachheit, mit der gemäß der Erfindung optimale, orale, feste Midodrin-Hydrochlorid-Retardformen realisiert werden können, war nicht vorhersehbar. Zieht man die Schwierigkeiten in Betracht, die bei der Retardierung von Midodrin-Hydrochlorid bisher bestanden haben und die bei den bisher bekannten biologisch abbaubaren Matrixformen in der Arzneiformengebung auftreten, so ist es für einen Fachmann ein unerwartetes Ergebnis, daß man im erfindungsgemäßen Matrixsystem durch einfaches Mischen und Verpressen feste Retardformen von Midodrin-Hydrochlorid mit nahezu beliebig variierbaren Freisetzungsraten herstellen kann. Einen weiteren unerwarteten Vorteil bieten die erfindungsgemäßen Retardformen aber auch dadurch, daß innerhalb der ersten 20 bis 60 Minuten nach der Varabreichung die zum Aufbau von therapeutisch wirksamen Blutspiegelwerten benötigte Initialdosis des Wirkstoffs direkt aus dem Matrixsystem freigesetzt wird. Dadurch kann bei den erfindungsgemäßen Präparaten im Gegensatz zu den meisten retardierten Arzneiformen auf zusätzliche Maßnahmen, die die Freisetzung der Initialdosis gewährleisten sollen, wie beispielsweise Aufsprühen, Beschichten oder Überziehen der Retardform mit dem Wirkstoff in Instantform, verzichtet werden. Auf diese Weise können die mit solchen Maßnahmen üblicherweise einhergehenden Verluste an Wirkstoff vermieden und das Herstellungsverfahren vereinfacht werden.

Die gleichmäßige und verzögerte Freisetzung des Wirkstoffs aus den erfindungsgemäßen Retardformen kann sowohl in Modellversuchen in vitro als auch in Tierversuchen in vivo nachgewiesen werden. So weist beispielsweise eine Midodrin-Hydrochlorid-Retardtablette gemäße der Erfindung, die 7.5 mg Gutron und 22.5 mg PHB mit einer Korngrößenfraktion von 0.2 bis 0.315 mm und einen Molekulargewicht von $1.06 \cdot 10^6$ in homogener Mischung enthält, im in vitro-Versuch nach einer Stunde eine Freisetzungsrate von 47.33%, entsprechend 3.5 mg Midodrin-Hydrochlorid auf, während nach 7 Stunden der Wirkstoff zu 100% aus der Retardform freigesetzt wird. Bei in vivo-Versuchen mit einer gleichen Midodrin-Hydrochlorid-Retardtablette erhält man ähnliche Freisetzungsraten, die beispielsweise 2

4

Stunden nach der Verabreichung 67.65% und 8 Stunden nach der Varabreichung 96.18% der in der Retardform anfänglich vorhanden Wirkstoffmenge betragen.

Die nachfolgenden Beispiel sollen das Wesen der Erfindung erläutern, ohne sie aber zu beschränken:

**Beispiel 1:**

| Rezeptur: | 1 Stück | Ansatz für 5 000 Stück |
|---|---|---|
| Midodrin-Hydrochlorid | 15.0 mg | 75 g |
| Poly-D(−-3-hydroxy-buttersäure (PHB) | 15.0 mg | 75 g |
| MG $1.06 \cdot 10^6$ | | |
| Korngrößenfraktion: 0.2—0.315 mm | | |

Verarbeitung:

Die Bestandteile werden gesiebt vermengt und in einem Kubusmischer homogenisiert.

Das Homogenat wird bei einem Preßdruck von 3.2 Tonnen/cm², entsprechend 313.92 N/mm², zu Tabletten verpreßt.

Tablettengewicht: 30 mg

Tablettengröße: Durchmesser 4.0 mm, Höhe 2.0 mm

Bruchfestigkeit: 9.8 kp

In vitro-Wirkstofffreigabe Midodrin-Hydrochlorid:

Bedingungen: U.S.P. XX-paddle-Methode, 100 Umdrehungen/Min
37°C, physiol. NaCl Lsg.

analyt. Bestimmung: direkt spektralphotometrisch bei Lambda=279 nm

Die Freigaben in vitro wurden in den folgenden Beispielen stets unter diesen Bedingungen durchgeführt:

| Stunde | 0.5 | 1 | 2 | 3 | 4 |
|---|---|---|---|---|---|
| Midodrin-Hydrochlorid | 33.66 | 55.88 | 91.92 | 99.42 | 100 |
| +/−<br>n=10 | 0.30 | 1.29 | 0.95 | 1.84 | — |

**Beispiel 2:**

| Rezeptur: | 1 Stück | Ansatz für 10 000 Stück |
|---|---|---|
| Midodrin-Hydrochlorid | 7.5 mg | 75 g |
| PHB | 22.5 mg | 225 g |
| MG $1.06 \cdot 10^6$ | | |
| Korngrößenfraktion: 0.315—0.4 mm | | |

Die Verarbeitung zu Tabletten erfolgt analog zu Beispiel 1.

Preßdruck: 3.2 Tonnen/cm², entsprechend 313.92 N/mm²

Tablettengewicht: 30 mg

Tablettengröße: Durchmesser 4.0 mm, Höhe 2.00 mm

Bruchfestigkeit: 10.4 kp

## EP 0 164 571 B1

In vitro-Wirkstofffreigabe Midodrin-Hydrochlorid:

| Stunde | 1 | 2 | 3 | 4 | 5 | 6 | hOL07 |
|---|---|---|---|---|---|---|---|
| Midodrin-Hydrochlorid | 47.33 | 75.30 | 81.40 | 91.30 | 95.60 | 97.33 | 100 |
| +/−<br>n=10 | 4.10 | 2.01 | 0.46 | 0.66 | 0.81 | 0.13 | — |

In vivo-Wirkstofffreigabe Midodrin-Hydrochlorid:

Bedingungen:

Die Tabletten werden Ratten mittels einer Spezialsonde peroral appliziert und die Ausstrommenge an Midodrin-Hydrochlorid zu den angegebenen Zeitpunkten (2, 4, 6, 8 Stunden) durch Rückstandsbestimmung ermittel. Dazu werden die Tiere mittels Nackenschlag getötet und die Tabletten aus dem Gastrointestinaltrakt entnommen. Nach Reinigung und Trocknung von Tabletten werden diese mechanisch zerkleinert, mit $H_2O$ versetzt und im Ultraschallbad zur vollständigen Freisetzung von Midodrin-Hydrochlorid weiterbehandelt. Nach dem Zentrifugieren wird Midodrin-Hydrochlorid direkt aus der Lösung bestimmt.

| Stunde | 2 | 4 | 6 | 8 |
|---|---|---|---|---|
| Midodrin-Hydrochlorid | 67.65 | 86.48 | 91.22 | 96.18 |
| +/−<br>n=6 | 0.93 | 1.46 | 1.73 | 1.03 |

Beispiel 3:

| Rezeptur: | 1 Stück | Ansatz für 10 000 Stück |
|---|---|---|
| Midodrin-Hydrochlorid | 7.5 mg | 75 g |
| PHB | 52.5 mg | 525 g |

MG $2.6 \cdot 10^5$
Korngrößenfraktion: 0.2—0.5 mm

Die Verarbeitung zu Tabletten erfolgt analog zu Beispiel 1 unter verschiedenen Preßdrucken.

Preßdruck: 0.5 Tonnen/cm$^2$      1.0, 1.5, 2.0 Tonnen
entsprechend 49.05 N/mm$^2$      entsprechend 98.1
147.15, 196.2 N/mm$^2$
Tablettengröße: Durchmesser 6.0 mm, Höhe 2,2 mm, Steghöhe 1.45 mm
Bruchfestigkeit: 13,0 kp      20 kp

In vitro-Wirkstofffreigabe Midodrin-Hydrochlorid:

| Stunde | 1 | 2 | 3 | 4 | 5 | 6 | 7 | 8 |
|---|---|---|---|---|---|---|---|---|
| Midodrin-Hydrochlorid | 20.6 | 29.28 | 39.08 | 47.99 | 54.35 | 67.37 | 68.53 | 72.06 |
| +/− | | 1.77 | 1.78 | 2.04 | 1.86 | 2.09 | 2.45 | 2.65 | 3.12 |

Die Freisetzungsraten sind vom Preßdruck im Bereich von 0.5 bis 2.0 Tonnen/cm$^2$, mit dem das Homogenat in Beispiel 3 zu Tabletten verpreßt wurde, unabhängig.

6

Beispiel 4:

| Rezeptur: | 1 Stück | Ansatz für 5 000 Stück |
|---|---|---|
| Midodrin-Hydrochlorid | 15.0 mg | 75 g |
| PHB | 45 mg | 225 g |

MG $6.2 \cdot 10^4$
Korngrößenfraktion: 0.2—0.4 mm

Die Verarbeitung zu Tabletten erfolgt analog zu Beispiel 1.

Preßdruck: 1.0—2.0 Tonnen/cm$^2$
entsprechend 98.1—196.2 N/mm$^2$
Tablettengewicht: 60 mg
Tablettengröße: Durchmesser 6.0 mm, Höhe 2,2 mm, Steghöhe 1.45 mm
Bruchfestigkeit: größer als 20 kp

In vitro-Wirkstofffreigabe Midodrin-Hydrochlorid:

| Stunde | 0.50 | 1 | 2 | 3 | 4 | 5 | 6 | 7 | 8 |
|---|---|---|---|---|---|---|---|---|---|
| Midodrin-Hydrochlorid | 30.0 | 39.55 | 53.16 | 65.58 | 72.60 | 80.72 | 87.87 | 94.40 | 100 |
| +/−<br>n=10 | 1.27 | 2.36 | 1.95 | 3.34 | 3.35 | 3.00 | 1.84 | 1.87 | — |

Beispiel 5:

| Rezeptur: | 1 Stück | Ansatz für 10 000 Stück |
|---|---|---|
| Midodrin-Hydrochlorid | 15.0 mg | 150 g |
| PHB | 75.0 mg | 750 g |

MG $1.46 \cdot 10^6$
Korngrößenfraktion: 0.2—0.315 mm

Die Verarbeitung zu Tabletten erfolgt analog zu Beispiel 1.

Preßdruck: 1.0—2.0 Tonnen/cm$^2$, entsprechend
98.1—196.2 N/mm$^2$
Tablettengewicht: 90 mg
Tablettengröße: Durchmesser 8.0 mm, Höhe 1.6 mm
Bruchfestigkeit: größer als 20 kp

In vitro-Wirkstofffreigabe Midodrin-Hydrochlorid:

| Stunde | 0.5 | 1 | 2 | 4 | 6 | 8 | 10 | 12 |
|---|---|---|---|---|---|---|---|---|
| Midodrin-Hydrochlorid | 21.28 | 32.22 | 44.26 | 64.43 | 78.33 | 86.34 | 91.52 | 95.02 |
| +/−<br>n=10 | 0.56 | 0.37 | 0.28 | 0.26 | 1.50 | 0.80 | 0.16 | 0.27 |

## EP 0 164 571 B1

### Beispiel 6:

| Rezeptur: | 1 Stück | Ansatz für 10 000 Stück |
|---|---|---|
| Midodrin-Hydrochlorid | 15.0 mg | 150 g |
| PHB | 105 mg | 1050 g |

MG $6.2 \cdot 10^4$
Korngrößenfraktion: 0.05—0.2 mm

Die Verarbeitung zu Tabletten efolgt analog zu Beispiel 1.

| | |
|---|---|
| Preßdruck: | 0.5 Tonnen/cm², entsprechend 49.05 N/mm² |
| Tablettengewicht: | 120 mg |
| Tablettengröße: | Durchmesser 8.0 mm, Höhe 1.6 mm |
| Bruchfestigkeit: | 13,0 kp |

In vitro-Wirkstofffreigabe Midodrin-Hydrochlorid:

| Stunde | 0.5 | 1 | 2 | 4 | 6 | 8 | 10 | 12 |
|---|---|---|---|---|---|---|---|---|
| Midodrin-Hydrochlorid | 18.02 | 23.73 | 32.39 | 45.57 | 53.71 | 61.93 | 69.77 | 77.87 |
| +/− n=10 | 0.85 | 1.17 | 1.03 | 1.58 | 1.79 | 2.38 | 2.83 | 3.56 |

### Beispiel 7:

| Rezeptur: | 1 Stück | Ansatz für 5 000 Stück |
|---|---|---|
| Midodrin-Hydrochlorid | 7.5 mg | 375 g |
| PHB | 52.5 mg | 265 g |

MG $2.6 \cdot 10^5$
Korngrößenfraktion: 0.2—0.315 mm

Die Verarbeitung zu Tabletten erfolgt analog zu Beispiel 1.

| | |
|---|---|
| Preßdruck: | 1.0 Tonnen/cm² entsprechend 98.1 N/mm² |
| Tablettengewicht: | 60 mg |
| Tablettengröße: | Durchmesser 6.0 mm, Höhe 2,1 mm, Steghöhe 1,6 mm |
| Bruchfestigkeit: | 15.8 kp |

In vitro-Wirkstofffreigabe Midodrin-Hydrochlorid:

| Stunde | 0.5 | 1 | 2 | 3 | 4 | 5 | 6 | 7 | 8 |
|---|---|---|---|---|---|---|---|---|---|
| Midodrin-Hydrochlorid | 30.32 | 40.85 | 54.74 | 64.27 | 71.05 | 78.76 | 82.06 | 84.06 | 90.89 |
| +/− n=10 | 1.06 | 0.41 | 0.06 | 0.82 | 0.39 | 0.45 | 0.63 | 0.26 | 0.34 |

# EP 0 164 571 B1

Beispiel 8:

| Rezeptur: | 1 Stück | Ansatz für 20 000 Stück |
|---|---|---|
| Midodrin-Hydrochlorid | 3.75 mg | 75 g |
| PHB | 56.25 mg | 1125 g |

MG $2.6 \cdot 10^5$
Korngrößenfraktion: 0.4—0.6 mm

Die Verarbeitung zu Tabletten erfolgt analog zu Beispiel 1.

Preßdruck: 1.0 Tonnen/cm², entsprechend 98.1 N/mm²
Tablettengewicht: 60 mg
Tablettengröße: Durchmesser 6.0 mm, Höhe 2.2 mm, Steghöhe 1.6 mm
Bruchfestigkeit: 15.8 kp

In vitro-Wirkstofffreigabe Midodrin-Hydrochlorid:

| Stunde | 0.5 | 1 | 2 | 3 | 4 | 5 | 6 | 7 | 8 |
|---|---|---|---|---|---|---|---|---|---|
| Midodrin-Hydrochlorid | 22.42 | 29.35 | 41.15 | 50.85 | 61.20 | 68.23 | 74.85 | 81.04 | 87.07 |
| +/−<br>n=10 | 0.31 | 0.09 | 0.84 | 0.16 | 0.25 | 0.00 | 0.50 | 0.23 | 0.46 |

**Patentansprüche für die Vertragsstaaten: BE CH DE FR GB IT LI LU NL SE**

1. Orale Retardformen des alpha-(2,5-Dimethoxy-phenyl)-beta-glycinamidoäthanols (Midodrin) in Form von festen, durch Verpressen hergestellten Arzneimittel-Formkörpern, bestehend aus dem Wirkstoff, einem polymeren Trägermaterial und gegebenenfalls in der Galenik üblichen Hilfs- und Zusatzstoffen, dadurch gekennzeichnet, daß die Retardform auf einen Gew.Teil alpha-(2,5-Dimethoxy-phenyl)-beta-glycinamidoäthanol in Form des Hydrochlorids (Midodrin-Hydrochlorid) 1 bis 15 Gew.Teile Poly-D(−)-3-hydroxybuttersäure mit einem Molekulargewicht von mindestens 50000 und einer ausgewählten Korngrößenfraktion im Bereich von 0.05 bis 0.6 mm in homogener Mischung enthält.

2. Retardformen gemäß Anspruch 1, dadurch gekennzeichnet, daß die Poly-D(−)-3-hydroxybuttersäure ein Molekulargewicht von 50000 bis 1 500 000 aufweist.

3. Retardformen gemäß Ansprüchen 1 und 2, dadurch gekennzeichnet, daß die Poly-D(−)-3-hydroxybuttersäure in der Mischung in einer Korngrößenfraktion im Bereich von 0.2 bis 0.5 mm vorliegt.

4. Retardformen gemäß Ansprüchen 1 bis 3, dadurch gekennzeichnet, daß sie auf 1 Gew.Teil Midodrin-Hydrochlorid 3 bis 5 Gew.Teile Poly-D(−)-3-hydroxybuttersäure enthalten.

5. Retardformen gemäß Ansprüchen 1 bis 4, dadurch gekennzeichnet, daß sie eine Gesamtwirkstoffdosis von 7.5 bis 15 mg Midodrin-Hydrochlorid pro Dosiseinheit enthalten.

6. Retardformen gemäß Ansprüchen 1 bis 5, dadurch gekennzeichnet, daß die Retardform eine Tablette ist, die gegebenenfalls eine oder mehrere Bruchrillen aufweist.

7. Verfahren zur Herstellung von oralen Midodrin-Retardformen gemäß den Ansprüchen 1 bis 6, dadurch gekennzeichnet, daß der Wirkstoff Midodrin-Hydrochlorid mit Poly-D(−)-3-hydroxybuttersäure mit einem Molekulargewicht von mindestens 50 000, die aus dem Fermentationsprodukt durch Mahlen und Siebanalyse in einer ausgewählten Korngrößenfraktion im Bereich von 0.05 bis 0.6 mm gewonnen wurde, im Gewichtsverhältnis von 1:1 bis 1:15 auf mechanische Weise vermischt und homogenisiert wird und gegebenenfalls zusätzlich mit in der Galenik üblichen Hilfs-und Zusatzstoffen, unter einem Druck von 0.1 bis 10 Tonnen/cm², entsprechend 9.81—981 N/mm², zu Arzneimittel-Formkörpern verpreßt wird.

8. Verfahren nach Anspruch 7, dadurch gekennzeichnet, daß das Homogenat aus Midodrin-Hydrochlorid und Poly-D(−)-3-hydroxybuttersäure direkt zu Arzneimittel-Formkörpern verpreßt wird.

9. Verfahren nach Anspruch 8, dadurch gekennzeichnet, daß das Homogenat zu Tabletten verpreßt wird.

**Patentansprüche für den Vertragsstaat: AT**

1. Verfahren zur Herstellung von oralen Retardformen des alpha-(2,5-Dimethoxy-phenyl)-beta-glycinamidoäthanols (Midodrin) in Form von festen, durch Verpressen hergestellten Arzneimittel-Formkörpern, bestehend aus dem Wirkstoff, einem polymeren Trägermaterial und gegebenenfalls in der Galenik üblichen Hilfs- und Zusatzstoffen, dadurch gekennzeichnet, daß man den Wirkstoff alpha-(2,5-Dimethoxy-phenyl)-beta-glycinamidoäthanol in Form des Hydrochlorids (Midodrin-Hydrochlorid) mit Poly-D(−)-3-hydroxybuttersäure mit einem Molekulargewicht von mindestens 50 000, die aus dem Fermentationsprodukt durch Mahlen und Siebanalyse in einer ausgewählten Korngrößenfraktion im Bereich von 0.05 bis 0.6 mm gewonnen wurde, im Gewichtsverhältnis von 1:1 bis 1:15 auf mechanische Weise vermischt, homogenisiert und gegebenenfalls unter Zusatz von in der Galenik üblichen Hilfs- und Zusatzstoffen unter einem Druck von 0.1 bis 10 Tonnen/cm², entsprechend 9.81 bis 981 N/mm², zu Arzneimittel-Formkörpern verpreßt.

2. Verfahren gemäß Anspruch 1, dadurch gekennzeichnet, daß man eine Poly-D(−)-3-hydroxybuttersäure mit einem Molekulargewicht von 50 000 bis 1 500 000 verwendet.

3. Verfahren gemäß Ansprüchen 1 und 2, dadurch gekennzeichnet, daß man die Poly-D(−)-3-hydroxybuttersäure in einer Korngrößenfraktion im Bereich von 0.2 bis 0.5 mm verwendet.

4. Verfahren gemäß Ansprüchen 1 bis 3, dadurch gekennzeichnet, daß man 1 Gew. Teil Midodrin-Hydrochlorid mit 3 bis 5 Gew. Teilen Poly-D(−)-3-hydroxybuttersäure zu einer homogenen Mischung verarbeitet.

5. Verfahren gemäß Ansprüchen 1 bis 4, dadurch gekennzeichnet, daß man Retardformen mit einer Gesamtwirkstoffdosis von 7.5 bis 15 mg Midodrin-Hydrochlorid pro Dosiseinheit herstellt.

6. Verfahren gemäß Ansprüchen 1 bis 5, dadurch gekennzeichnet, daß man die homogene Mischung aus Midodrin-Hydrochlorid und Poly-D(−)-3-hydroxybuttersäure direkt zu Arzneimittel-Formkörpern verpreßt.

7. Verfahren gemäß Ansprüchen 1 bis 5, dadurch gekennzeichnet, daß man die homogene Mischung aus Midodrin-Hydrochlorid und Poly-D(−)-3-hydroxybuttersäure zu Tabletten verpreßt, die gegebenenfalls eine oder mehrere Bruchrillen aufweisen.

**Revendications pour les Etats contractants: BE CH DE FR GB IT LI LU NL SE**

1. Formules retard orales de l'alpha-(2,5-diméthoxyphénol)-béta-glycinamidoéthanol (Midodrine) sous forme de grains moulés durs médicamenteux, préparés par compression, comprenant le principe actif, un composé support polymère et le cas échéant des substances d'additions ou vectrices classiques dans le processus galénique, caractérisées en ce qu'elles renferment un mélange homogène d'une partie en poids de l'alpha-(2,5-diméthoxyphényl)-béta-glycinamidoéthanol sous forme d'hydrochlorure (hydrochlorure de Midodrine), de 1 à 15 parties en poids d'acide butyrique poly-D(−)-3-hydroxy ayant un poids moléculaire d'au moins 50 000 et une granulation choisie entre 0,05 et 0,6 mm.

2. Formules retard selon la revendication 1, caractérisées en ce que l'acide butyrique poly-D(−)-3-hydroxy à un poids moléculaire compris entre 50 000 et 1 500 000.

3. Formules retard selon la revendication 1 ou 2, caractérisées en ce que l'acide butyrique poly-D(−)-3-hydroxy présente dans le mélange une granulation comprise entre 0,2 et 0,5 mm.

4. Formules retard selon les revendications 1 à 3, caractérisées en ce qu'elles renferment 1 partie en poids d'hydrochlorure de Midodrine et 3 à 5 parties en poids d'acide butyrique poly-D(−)-3-hydroxy.

5. Formules retard selon les revendications 1 à 4, caractérisées en ce qu'elles renferment 7,5 à 15 mg de principe actif hydrochlorure de Midodrine par unité de dose.

6. Formules retard selon les revendications 1 à 5, caractérisées en ce qu'elles se présentent sous forme de comprisés comportant une ou plusieurs lignes de rupture.

7. Procédé de préparation de formules retard orales selon les revendications 1 à 6, caractérisé en ce qu'on mélange mécaniquement la substance active hydrochlorure de Midodrine avec l'acide butyrique poly-D(−)-3-hydroxy ayant une masse moléculaire d'au moins 50 000 amené à une granulation de 0,05 à 0,6 mm par concassage et tamisage, à partir d'un produit de fermentation, dans un rapport massique de 1:1 à 1:15, avec addition éventuelle de substances d'addition et vectrices classiques dans le processus galénique, et en ce qu'on comprime sous forme de grains moulés médicamenteux à une pression de 0,1 à 10 tonnes/cm², correspondant à 9,81—981 N/mm².

8. Procédé selon la revendication 7, caractérisé en ce que la phase homogène d'hydrochlorure de Midodrine et d'acide butyrique poly-D(−)-3-hydroxy est comprimée directement sous forme de grains moulés médicamenteux.

9. Procédé selon la revendication 8, caractérisée en ce que la phase homogène est compressée sous forme de comprimés.

**Revendications pour l'Etat contractant: AT**

1. Procédé de préparation de formules retard orales de l'alpha-(2,5-dimethoxy-phényl)-béta-glycinamidoéthanol (Midodrine) sous forme de grains moulés durs médicamenteux, préparés par compression, comprenant le principe actif, un composé support polymère et le cas échéant des substances d'addition ou vectrices classiques dans le processus galénique, caractérisé en ce qu'on mélange mécaniquement 1 partie en masse de principe actif, l'alpha-(2,5-diméthoxy-phényl)-béta-glycinamidoéthanol, sous forme d'hydrochlorure (hydrochlorure de Midodrine), avec 1 à 15 parties en masse d'acide butyrique poly-D(−)-3-hydroxy ayant une masse moléculaire d'au moins 50000, obtenu à partir d'un produit de fermentation par broyage et tamisage, à une granulation choisie entre 0,05 et 0,6 mm, et en ce qu'on homogénéise et on comprime, après addition le cas échéant de substances d'additions ou vectrices classiques dans le processus galénique, en grains moulés médicamenteux sous une pression de 0,1 à 10 tonnes/cm$^2$, correspondant à 9,81—981 N/mm$^2$.

2. Procédé selon la revendication 1, caractérisé en ce qu'on utilise un acide butyrique poly-D(−)-3-hydroxy ayant une masse moléculaire comprise entre 50 000 et 1 500 000.

3. Procédé selon la revendication 1 ou 2, caractérisé en ce qu'on utilise un acide butyrique poly-D(−)-3-hydroxy présentant une granulation comprise entre 0,2 et 0,5 mm.

4. Procédé selon les revendications 1 à 3, caractérisées en ce qu'on réalise un mélange homogène de 1 partie en masse d'hydrochlorure de Midodrine et de 3 à 5 parties en masse d'acide butyrique poly-D(−)-3-hydroxy.

5. Procédé selon les revendications 1 à 4, caractérisé en ce qu'on prépare une formule retard comprenant 7,5 à 15 mg de principe actif hydrochlorure de Midodrine par unité de dose.

6. Procédé selon les revendications 1 à 5, caractérisé en ce qu'on comprime le mélange homogène d'hydrochlorure de Midodrine et d'acide butyrique poly-D(−)-3-hydroxy directement sous forme de comprimés médicamenteux.

7. Procédé selon les revendications 1 à 6, caractérisé en ce qu'on comprime le mélange homogène d'hydrochlorure de Midodrine avec l'acide butyrique poly-D(−)-3-hydroxy directement en comprimés, comportant éventuellement une ou plusieurs lignes de rupture.

**Claims for the Contracting States: BE CH DE FR GB IT LI LU NL SE**

1. Oral sustained-release forms of alpha-(2,5-dimethoxyphenyl)-beta-glycinamidoethanol (midodrin) in the form of solid shaped pharmaceutical articles, produced by compressing, and consisting of the active compound, a polymeric support material and, if appropriate, auxiliaries and additives customary in pharmaceutical formulation, characterized in that the sustained-release form contains 1 to 15 parts by weight of poly-D(−)-3-hydroxybutyric acid having a molecular weight of at least 50,000 and a selected particle size fraction in the range from 0.05 to 0.6 mm to one part by weight of alpha-(2,5-dimethoxyphenyl)-beta-glycinamidoethanol in the form of the hydrochloride (midodrin hydrochloride) in a homogeneous mixture.

2. Sustained-release forms according to Claim 1, characterized in that the poly-D(−)-3-hydroxybutyric acid has a molecular weight of 50,000 to 1,500,000.

3. Sustained-release forms according to Claims 1 and 2, characterized in that the poly-D(−)-3-hydroxybutyric acid is present in the mixture in a particle size fraction in the range from 0.2 to 0.5 mm.

4. Sustained-release forms according to Claims 1 to 3, characterized in that they contain 3 to 5 parts by weight of poly-D(−)-3-hydroxybutyric acid to 1 part by weight of midodrin hydrochloride.

5. Sustained-release forms according to Claims 1 to 4, characterized in that they contain a total active compound dose of 7.5 to 15 mg of midodrin hydrochloride per dose unit.

6. Sustained-release forms according to Claims 1 to 5, characterized in that the sustained-release form is a tablet which optionally has one or more breaking notches.

7. Process for the preparation of oral midodrin sustained-release forms according to Claims 1 to 6, characterized in that the active compound midodrin hydrochloride is mechanically mixed and homogenized in a weight ratio of 1:1 to 1:15 with poly-D(−)-3-hydroxybutyric acid having a molecular weight of at least 50,000, which has been obtained from the fermentation product in a selected particle size fraction in the range from 0.05 to 0.6 mm by grinding and sieve analysis and is compressed to give shaped pharmaceutical articles, if appropriate using auxiliaries and additives customary in pharmaceutical formulation, under a pressure of 0.1 to 10 tonnes/cm$^2$, corresponding to 9.81—981 N/mm$^2$.

8. Process according to Claim 7, characterized in that the homogenate of midodrin hydrochloride and poly-D(−)-3-hydroxybutyric acid is compressed directly to give shaped pharmaceutical articles.

9. Process according to Claim 8, characterized in that the homogenate is compressed to give tablets.

**Claims for the Contracting State: AT**

1. Process for the preparation of oral sustained-release forms of alpha-(2,5-dimethoxyphenyl)-beta-glycinamidoethanol (midodrin) in the form of solid shaped pharmaceutical articles, produced by compressing, and consisting of the active compound, a polymeric support material and, if appropriate, auxiliaries and additives customary in pharmaceutical formulation, characterized in that the active compound alpha-(2,5-dimethoxyphenyl)beta-glycinamidoethanol in the form of the hydrochloride (midodrin hydrochloride) is mechanically mixed in a weight ratio of 1:1 to 1:15 with poly-D(−)-3-hydroxybutyric acid having a molecular weight of at least 50,000, which has been obtained from the fermentation product in a selected particle size fraction in the range from 0.05 to 0.6 mm by grinding and sieve analysis, homogenized and compressed to give shaped pharmaceutical articles, if appropriate with the addition of auxiliaries and additives customary in pharmaceutical formulation, under a pressure of 0.1 to 10 tonnes/cm$^2$, corresponding to 9.81 to 981 N/mm$^2$.

## EP 0 164 571 B1

2. Process according to Claim 1, characterized in that a poly-D(−)-3-hydroxybutyric acid having a molecular weight of 50,000 to 1,500,000 is used.

3. Process according to Claims 1 and 2, characterized in that the poly-D(−)-3-hydroxybutyric acid is used in a particle size fraction in the range from 0.2 to 0.5 mm.

4. Process according to Claims 1 to 3, characterized in that one part by weight of midodrin hydrochloride is processed with 3 to 5 parts by weight of poly-D(−)-3-hydroxybutyric acid to give a homogeneous mixture.

5. Process according to Claims 1 to 4, characterized in that sustained-release forms are prepared having a total active compound dose of 7.5 to 15 mg of midodrin hydrochloride per dose unit.

6. Process according to Claims 1 to 5, characterized in that the homogeneous mixture of midodrin hydrochloride and poly-D(−)-3-hydroxybutyric acid is compressed directly to give shaped pharmaceutical articles.

7. Process according to Claims 1 to 5, characterized in that the homogeneous mixture of midodrin hydrochloride and poly-D(−)-3-hydroxybutyric acid is comprssed to give tablets which optionally have one or more breaking notches.

12